# EUROPEAN PATENT APPLICATION

(11) **EP 3 357 420 A1**
(43) Date of publication of application: **08.08.2018**
(21) Application number: 17155077.5
(22) Date of filing: 07.02.2017
(51) Int. Cl.: A61B 5/11, A61B 5/00

(54) **MONITORING ACTIVITY OF A PERSON**

(71) Applicant: Koninklijke Philips N.V., 5656 AE Eindhoven (NL)
(72) Inventor: VAN DE WOUW, Doortje, 5656 AE Eindhoven (NL); AARTS, Ronaldus Maria, 5656 AE Eindhoven (NL); TEN KATE, Warner Rudolph Theophile, 5656 AE Eindhoven (NL)
(74) Representative: de Haan, Poul Erik

(57) **Abstract**

Presented are concepts for monitoring a physical or mental capability of a person. One such concept employs signals from sensors that detect the presence of a person within first and second zones. A value of time elapsed between a change in detected presence indicated by the sensor signals may be used to determine a transition time of the person. Information about a transition time of the person may, for example, be useful for the purpose of assessing the person's physical and/or mental abilities.

## Description

### FIELD OF THE INVENTION

This invention relates to monitoring activity of a person.

### BACKGROUND OF THE INVENTION

Functional assessment or monitoring of a person's health status, physical abilities, mental abilities, or recuperation after injury, hospitalization and treatment is of primary concern in most branches of medicine, including geriatrics, rehabilitation and physical therapy, neurology and orthopaedics, nursing and elder care.

Investigations have found that an individual's functional ability is actually environment-specific, since function increases when subjects are in familiar surroundings due to reduced confusion. Also, one-time assessment of function does not allow for assessment of variability of functional performance over the course of a day or several days, nor does it allow for assessment of change which is important in determining the adequacy of certain clinical services and treatments (such as rehabilitation) following functional loss.

A consensus therefore exists that it is preferable to assess or monitor independent functioning of a person at their home or within familiar surroundings.

A level of independent function is commonly indicated by the quality in which Activities of Daily Living (ADLs) are performed. ADLs refer to the most common activities that people perform during a day. Therefore, a reduced quality in the ADLs can be an indicator for care needed. For example, an anomaly in the regular performance of one or more ADLs can serve as warning for special attention.

Devices and systems have been developed to monitor the ADLs of individuals as they live independently in their own home or within familiar surroundings. For example, one such known system for detecting activities of daily living of a person system comprises three main components: (i) a sensor system that collects information about the person's activities and behaviours; (ii) an intelligence (or information processing) system that interprets the sensor signals for determination of ADL behaviour; and (iii) a user interface system that enables care givers to inspect the interpreted (processed) information. The intelligence system typically makes use of computational techniques known in the art as artificial intelligence. The system may be supported by conventional technologies for data collection, transmission, and storage.

In practice, however, a major difficulty is encountered by the wide range of variations that can happen in actual care cases. Since there are so many possible circumstances, situations, environment layouts and contexts that can occur in daily life, it is common to employ numerous sensors in an attempt to capture enough information about a person's activities and/or environment to enable identification of specific activities. This, however, typically increases complexity and/or costs and may therefore be undesirable.

Also, a monitored person's physical and/or mental abilities may vary (e.g. decline or reduce) over time. As a result, it can be difficult to determine when some form of assistance, help of supervision may be needed.

### SUMMARY OF THE INVENTION

The invention aims to at least partly fulfil the aforementioned needs. To this end, the invention provides devices, systems and methods as defined in the independent claims. The dependent claims provide advantageous embodiments.

There is provided a system for monitoring an activity of daily living of a person, wherein the system comprises: a signal interface adapted to receive: a first sensor output signal representative of detected presence of a person within a first zone; and a second sensor output signal representative of detected presence of a person within a second zone; a signal processing unit adapted to determine a value of time elapsed between a change in detected presence represented by the first sensor output signal and a change in detected presence represented by the second sensor output signal; and a monitor unit adapted to determine a current value of a transition time of the person based on the determined value of time.

Embodiments may be based on the insight that a measure or metric of a person (which may, for example, be representative of speed of movement (e.g. walking speed)) may be determined using two relatively simple presence detectors (e.g. PIR sensors) that are separated so as to sense the presence of a person into two zones. By determining the time elapsed between activity of the two sensors, a transition time (i.e. a time taken for the person to travel between two zones) may be determined. Also, from this, a speed at which a person travelled between the two zones may be calculated (since the distance travelled by the person may be known). A walking speed of a person may therefore be calculated or inferred from the timings of ON/OFF triggering of two spaced apart sensors. For example, as monitored person leaves a first zone (e.g. Zone A) monitored by a first sensor, the first sensor's output signal may switch/transition from ON (or 'HIGH','Logical 1', etc.) to OFF (or 'LOW', 'Logical 0', etc.). Then, as the monitored person enters a second zone (e.g. Zone B) monitored by a second sensors, the second sensor's output signal may switch/transition from OFF (or 'LOW', 'Logical 0', etc.) to ON (or 'HIGH','Logical 1', etc.). The timing elapsed between such switching/transitions may be determined and then used to calculate a transition time, which may then be used to calculate a travel speed of the person (based on a known or assumed distance between the first and second zones). Where a distance between zones is not known or assumed, a transition time (i.e. time taken for the person to transition between zone/areas/locations) may be established from the timing elapsed between switching of the sensor output signal. Such transition time may be useful as a general metric for monitoring a person.

Also, depending on configuration of the sensors and the direction in which a transition is defined, the transition time may have a positive or negative value. A direction of travel may thus be inferred from whether the transition time has a positive or negative value for example.

Information about a transition time or walking speed of a monitored person may be useful for the purpose of assessing the person's physical and/or mental abilities. For example, walking speed has been reported to be a general/summary indicator of health, wherein a relatively slow walking speed (or declining walking speed) may be indicative of mental or physical issues.

Thus, rather than employing numerous and/or complex sensor arrangements and attempting to process complicated sensor data, proposed embodiments may use simple on/off or binary signals from two spaced-apart presence sensors (and such sensors may already be present for other purposes including intruder detection or other forms of ADL monitoring for example). Embodiments may therefore be implemented in conjunction with pre-existing, pre-installed or otherwise separately-provisioned presence sensors, and the output signals from such sensors may be received and processed in accordance with proposed concepts. Other embodiments may be provided with sensors (e.g. where appropriate presence sensors are not already available). For instance, a system according to an embodiment may comprise: a first sensor adapted to detect the presence of a person within the first zone and to generate the first sensor output signal representative of the detected presence; a second sensor adapted to detect the presence of a person within the second zone and to generate the second sensor output signal representative of the detected presence. By way of example, each of the first and second sensors may comprise at least one of: a camera; a radar system; a WiFi signal detection system; a pyroelectric infrared sensor; a weight sensor; and a pressure sensor

Based on the timing of changes, switches or transitions in the sensor signals, embodiments may determine (e.g. infer) information about a transition speed or speed of movement (e.g. walking speed) of a person without relying on complex and/or expensive sensor arrangements, thus reducing costs.

Information regarding the physical and/or mental health of a person may be inferred from walking speed values obtained from two presence detectors/sensors (which may, for example, be strategically arranged in adjacent rooms of the person's house). For instance, a first sensor may be positioned so as to detect the presence of a person in a living room, and a second sensor may be positioned so as to detect the presence of a person in a dining room (which may be adjacent to the living room). As the person walks from the living room to the dining room, the time elapsed between: (i) the signal from the first sensor indicating a change in detected presence in the living room (i.e. from detected presence to no detected presence); and (ii) the signal from the second sensor indicating a change in detected presence in the dining room (i.e. from no detected presence to detected presence may be determined. Based on the determined value for the time elapsed and a known (or assumed) distance between the monitored zones/areas of the first and second sensors, the walking speed of the person may be calculated. In this way, no additional sensors and/or monitors (such a light switch monitors, or light sensors, etc.) may be required in order to determine and monitor the speed of the person. Also, in some embodiments it may not be required to know or assume the distance between monitored zones/areas. Instead, it may suffice to simply observe the time elapsed (or transition time) since such a time value may be monitored in order to detect a variation (e.g. increase or decrease in transition time) and from which a change in walking speed may be inferred. This may also provide the advantage of reduced complexity, since no estimators are needed in order to determine at an accurate value of walking speed for example.

Embodiments may thus provide information on the movement speed of a person in a non-intrusive manner (e.g. without requiring a person to wear, operate or otherwise employ a sensor such as an accelerometer).

Embodiments may also be extended to providing information on the transition times for several room/area transitions, including in the reverse direction. A matrix or table of transition times may therefore be built, and this may be used to monitor transition time between multiple zones or areas with improved accuracy and/or flexibility.

Each of the first and second sensor output signals may comprise a binary signal indicating either detected presence or detected non-presence of the person. For example, a sensor may simply comprise a signal which is either ON or OFF depending on whether or not the sensors detects the presence of a person within its monitored area/zone. In other examples, the signal may comprise a signal or data field which has two possible values (such as a HIGH/LOW or '0/1' for example) indicative of detected presence or non-presence of a person within its monitored area/zone. The signal processing unit may then be adapted to determine the value of time (e.g. transition time) by determining the time elapsed between a change in the binary signal of first sensor output signal and a change in the binary signal of the second sensor output signal. For this purpose, the signal may comprise a timestamp or other time-indicative value which may be used to indicate the associated time at which the presence or non-presence is detected. This may have the advantage of catering for any delays in transmission or reception of the signals from the sensors.

Alternatively, or additionally, the value of time elapsed between a change in detected presence by the first and second sensors may be determined based on the times at which the signals from the first and second signals were transmitted or received (e.g. where the signals are transmitted immediately upon detecting the presence of a person or immediately upon a change in detected presence). This approach may avoid a need for the signals to include time information, and thus avoid the need for a clock or other timing arrangement to be implemented by the sensors.

Some embodiments may further comprise a monitor unit adapted to determine a trend in the transition time of the person based on the current value of the transition time of the person and historical data relating to one or more previously determined values of the transition time of the person.

Thus, there may be proposed a concept of monitoring a physical or mental capability of a person by identifying a trend in the person's transition time (from which speed may be inferred for example). By using a currently sensed or determined transition time or speed, along with one or more previously determined values of transition time or speed, a trend, change or drift of the transition time or speed (or a property thereof) may be identified. For example, by detecting a consistent change or pattern in detected speed values over time, a trend in a person's speed (and thus physical or mental capability) may be inferred and, from this, current and/or future care/help/assistance requirements may be determined. Thus, there may be proposed a concept of determining a trend in a person's capability or activity based on current and historical speed. For instance, a determined walking speed of the person may be used to infer a physical or mental capability of a person, and this may be undertaken numerous times over an extended time period (such as hours, days, weeks, months or years) so as to enable monitoring of the person's physical or mental capability over time. Furthermore, detected walking for a predetermined timeframe (e.g. a week or month) may be used to estimate a trend in a physical or mental capability of the person for a current, following and/or preceding timeframe.

A trend in the physical and/or mental capability of a person may therefore be inferred without employing a complex and/or expensive sensor arrangement within a monitored environment. This may help to reduce associated cost and/or complexity of a monitoring system. For example, embodiments may alleviate a need to arrange the sensors in dedicated or exact locations of the sensors so that they have specific inter-sensor distances. For instance, rather than focussing on obtaining an exact or accurate speed measurement for a single transition between two zones, embodiments may instead observe a trend or pattern in the transition times (and thus the corresponding walking speed).

For example, a trend of declining/decreasing velocity or speed of movement of the person (as inferred from timings between changes in signals issued by two spaced apart presence sensors) may be used to infer and monitor the person's physical speed, and may further identify a future point in time where the person's physical speed declines to a point where assistance or help will be required. Predictive analytics may be employed to try and identify (and thus prevent or avoid) undesirable or worst case outcomes. This may help to provide cost savings (e.g. in a health care system) by timely enabling intervention. Embodiments may therefore be useful for identifying future requirements of a monitored person, and this may be achieved via extrapolation of a trend determined by a proposed embodiment.

Embodiments may therefore enable the monitoring of a person's physical capability by analysing the elapsed time between sensor activations (e.g. time to travel between two monitored areas or rooms) and inferring a trend from detected changes in the elapsed time values.

In some embodiments, the monitor unit may be further adapted to detect an irregularity or anomaly in the determined trend. For example, the monitor unit may be adapted to detect an irregularity based on a comparison of the determined trend with a threshold value. For example, this may provide the advantage that an alarm may be given if the person takes too long to walk between two rooms indicating that, for example, the person has become unwell or fallen over. It may also provide for the identification of different persons or pets so that they do not interfere with the data or trend of the monitored person. By way of example, in some embodiments, the threshold value may be determined based on at least one of: one or more previously detected values of the speed of the person; and one or more previously determined changes in values of the speed of the person.

To detect an irregularity or anomaly, the monitor unit may undertake a comparison of the determined trend with a threshold value. For example, the monitor unit may employ a data processing unit that compares the determined trend with a threshold value and then generates an alert signal if the determined trend exceeds the threshold value. The threshold may be pre-programmed and fixed, but it may be preferable to enable the threshold value to be set by a user preference. Also, the threshold value may relate to a future value, and the trend may be extrapolated for comparison of an extrapolated value with the future threshold value. This may identify when a threshold value may be exceeded in the future, for example. More sophisticated solutions are known as change point detection, of which the CUSUM algorithm represent a popular detection principle.

In an embodiment, the monitor unit may be further adapted to calculate an estimated future value based on the determined trend, and the monitor unit may be further arranged to detect an irregularity based on a comparison of the estimated future value with a threshold value and to generate an alert output signal in response to the detected irregularity.

Embodiments may further comprise a user input interface adapted to receive a user input for defining or modifying one or more alert conditions, and the monitor unit may be adapted to generate an alert output signal based on the determined trend and the one or more alert conditions.

Further, the threshold may be based on previously determined value of the transition time of the person and/or a previously determined trend in the transition time of the person. For instance, the threshold value may be determined based on at least one of: one or more previously detected values of the transition time of the person; and one or more previously determined changes in values of the transition time of the person. In other words, the threshold may be defined by taking account of a history of detected values and/or a history of changes in values of the transition time so that it can be used to identify outlying values or anomalies (e.g. caused by a different person or pet).

In a further embodiment of the system the monitor unit may be further adapted to store the determined transition time of the person in a database. Previously determined values may therefore be stored, in a historical database for example, and then used in subsequent calculations. Furthermore, a currently detected value of transition time may be used to re-calculate or refine a previously determined trend.

Thus, a pattern of the transition time of the person may be stored. Shifts in this pattern may indicate that the person is in need of help. For example, the person may start to walk slower than normal due to increased physical difficulties, and this may be inferred from a trend in transition time of the monitored person.

The monitor unit may be further adapted to detect an irregularity or anomaly in the database. For example, by adding time/date information to the determined transition time values, the duration between the determined values may be determined. With the time/date information, the frequency of the movement may be determined. For example, the frequency of the movement activity from Room A to Room B may be 'three times a day', or '21 times a week'. An irregularity in the activity database may then, for example, be that a frequency of the movement from Room A to Room B has decreased significantly. For example, the average number of times movement from Room A to Room B occurs may be determined using the data from the activity profile. When the number of occurrences of movement from Room A to Room B is less the average this indicates an irregularity.

While this may provide an indicator by itself, it may also affect the confidence interval of the measured (average) transition speed. More measurements may provide a more reliable estimate (the intrinsic variation in the transition action itself will remain). Therefore, a minimum number of transition may be required before a (average) value will be used. A model may be that every day an average is determined for all possible room transitions, and only those that are derived from a sufficient number (for all in the whole series) may be used in testing for a change. Alternatively, the spread (or other metric for scale) may be determined for every day, and that value may be used to decide whether or not to use the corresponding transition times. Note that the distribution of transition times may in general be skewed; so there is a left and a right "spread".) Also, a transition from zone 0 to zone 1 may be treated as a different series from a transition from zone 1 into zone 0, for example. Selection of transition times may also be based on context, such as time of day, or having been sleeping, or returning/leaving home, etc.

In a further embodiment, the monitor unit may be further arranged to generate a warning signal in response to a detected irregularity. The irregularity may indicate that the person is in need of help and/or advise the person to start doing exercises to improve physical condition for example. In a further example, a medical practitioner, a caregiver, a family member or close relative may be advised by the system (using the alert signal) to pay a visit to the person. In a further embodiment the warning signal may be provided to the person himself/herself. For example, the warning signal may be a feedback signal advising the person to take a certain medication.

Embodiments may be adapted to provide a generated alert or warning signal to at least one of: the person; a medical practitioner; and a caregiver.

Embodiments may further comprise a control unit adapted to generate a control signal for controlling an item or object based on the determined transition time of the user. In this way, embodiments may be adapted to provide control signals that are suitable for use by the control system, thereby enabling embodiments to modify or control operation of the control system (and thus the object(s) controlled by the control system). For example, beyond monitoring, embodiments may also control objects/devices based on the obtained speed data. This may be done directly, by generating and communicating a control signal to an object so as to control (e.g. change, modify or adapt) its operation, or done indirectly by communicating a signal to a control system so as to cause the control system to control the object in response. In this way, operation of an object (such as a light) may be adapted based on the obtained control data (e.g. to alter the lighting conditions).

By way of example, the object may comprise a lighting device or home appliance adapted to be used for the execution of an ADL. Also, the control system may be a smart-home control system adapted to control one or more operations of a light and/home application within the smart-home. A lighting device or home appliance may therefore be automatically switched on or off at an appropriate time, and the time may be dependent on the detected transition time and direction of the person.

An embodiment may be further adapted to generate a display control signal for modifying a graphical element based on the determined transition time of the person, and the system may further comprise: a display system adapted to display the graphical element in accordance with the generated display control signal. In this way, a user (such as a care giver) may have an appropriately arranged display system that can receive and display information about a determined transition time or speed of the monitored person/patient, and that person/patient may be remotely located from the user. Embodiments may therefore enable a user to remotely monitor a person using a portable display device, such as a laptop, tablet computer, mobile phone, PDA, etc.

It will be appreciated that all or part of a system according to an embodiment may comprise one or more data/signal processing units. For example, the signal processing unit may be implemented using a single processor which is adapted to undertake data processing in order to determine a transition time of the person (based on the timing of signals from two presence sensors). The signal processing unit may be remotely located from the sensors, and signals issued by the sensors may be communicated to the signal processing unit via respective communication links.

The system may further comprise: a server device comprising the signal processing unit. Dedicated data/signal processing means may therefore be employed for the purpose of determining a transition time of the person, thus reducing processing requirements or capabilities of other components or devices of the system.

The system may further comprise a client device, wherein the client device comprises the signal processing unit and a display system. In other words, a user (such as a care giver) may have an appropriately arranged client device (such as a laptop, tablet computer, mobile phone, PDA, etc.) which processes received sensor signals in order to determine a transition time of the person.

Thus, processing may be hosted at a different location from where the sensor signals are generated or issued. For example, for reasons of power efficiency (e.g. to improve battery lifetime) it might be advantageous to execute only part of the processing at the sensor location(s), thereby reducing associated costs, processing power, transmission requirements, etc.

Embodiments may also enable some of the processing load to be distributed throughout the system. For example, pre-processing may be undertaken at a sensor system. Alternatively, or additionally, processing could be undertaken at a communication gateway. In some embodiments, processing may be undertaken at a remote gateway or sever, thus relinquishing processing requirements from an end-user or output device. Such distribution of processing and/or hardware may allow for improved maintenance abilities (e.g. by centralising complex or expensive hardware in a preferred location). It may also enable computational load and/or traffic to be designed or located within a networked system according to the processing capabilities available. A preferable approach may be to process sensor signals locally and transmit extracted data for full processing at a remote server.

Thus, it will be understood that processing capabilities may be distributed throughout the system in different ways according to predetermined constraints and/or availability of processing resources.

According to another aspect of the invention, there is provided a method for monitoring activity of a person, wherein the method comprises: detecting, with a first sensor, the presence of a person within a first zone and generating a first sensor output signal representative of the detected presence; detecting, with a second sensor, the presence of a person within a second zone and generating a second sensor output signal representative of the detected presence; determining a value of time elapsed between a change in detected presence represented by the first sensor output signal and a change in detected presence represented by the second sensor output signal; and determining a current value of the transition time of the person based on the determined value of time.

The non-intrusive character of the monitoring a person's transition time, movement or walking speed may be realized by analysing the timing of changes or transitions in signals from two presence sensors. A change or transition in a signal from a first sensor may for example indicate a person is no longer present in a first zone/area monitored by the first sensor. This may then be coupled with a change or transition in a signal from a second sensor indicating a person has entered a second zone/area monitored by the second sensor. The time elapsed between the indication of the person leaving the first zone and entering the second zone may be analysed in conjunction with a known (or assumed) distance between the zone boundaries in order to calculate a speed of the person.

In an embodiment, the step of determining the value of time may therefore comprise determining the time elapsed between a change in the binary signal of first sensor output signal and a change in the binary signal of the second sensor output signal.

In a further embodiment, the method may further comprise the step of: determining a trend in the physical or mental capability of the person based on the current value of the transition time of the person and historical data relating to one or more previously determined values of the transition time of the person. Embodiments may also further comprise: detecting an irregularity in the determined trend; and generating an alert output signal in response to the detected irregularity.

According to yet another aspect of the invention, there is provided a computer program product for an activity of daily living of a person, wherein the computer program product comprises a computer-readable storage medium having computer-readable program code embodied therewith, the computer-readable program code configured to perform all of the steps of a proposed embodiment.

In an embodiment, a computer system may be provided which comprises: a computer program product according to an embodiment; and one or more processors adapted to perform a method according to an embodiment by execution of the computer-readable program code of said computer program product.

In a further aspect the invention relates to a computer-readable non-transitory storage medium comprising instructions which, when executed by a processing device, execute the steps of a method for monitoring an activity of daily living of a person according to an embodiment.

These and other aspects of the invention will be apparent from and elucidated with reference to the embodiment(s) described hereinafter.

### BRIEF DESCRIPTION OF THE DRAWINGS

Examples in accordance with aspects of the invention will now be described in detail with reference to the accompanying drawings, in which:
Figure 1A is a simplified block diagram of a system for monitoring ADLs of a person within an environment according to an embodiment;
Figure 1B illustrates an exemplary implementation of the embodiment of Fig. 1A, wherein PIR sensors are implemented to detect the presence of person in first and second adjacent rooms;
Figure 1C illustrates a graph depicting transition time (t) against transition instance for a normal variation/trend (depicted by the line marked with triangles) and for a trend of declining speed (depicted by the line marked with circles).
Figure 2 is a simplified block diagram of a system for monitoring ADLs of a person within an environment according to another embodiment;
Figure 3 is a flow diagram of a method for monitoring ADLs of a person within an environment according to an embodiment; and
Figure 4 is a simplified block diagram of a computer within which one or more parts of an embodiment may be employed.

### DETAILED DESCRIPTION OF THE EMBODIMENTS

Proposed is a concept for monitoring a person within an environment, which may be useful for the purpose of unobtrusively monitoring the well-being of the person for example. Such persons may, for instance, include a disabled person, an elderly person, an injured person, a medical patient, etc. Elderly persons can mean persons above 50 years, above 65 years, above 70, or above 80 years old, for example.

Illustrative embodiments may be utilized in many different types of monitoring environments, such as a hospital, ward, care home, person's home, etc. In order to provide a context for the description of elements and functionality of the illustrative embodiments, the Figures are provided hereafter as examples of how aspects of the illustrative embodiments may be implemented. It should therefore be appreciated the Figures are only examples and are not intended to assert or imply any limitation with regard to the environments, systems or methods in which aspects or embodiments of the present invention may be implemented.

It has been recognized that, in many care situations, there is often a need to be informed about the ADLs a person is performing. There may also be a need to be alerted when an anomaly occurs. The type of anomaly can be different per case. A large class of anomalies relate to aberrations in an ADL routine of the person. For example, an above average number of toilet visits during the night. More severe incidents form another class, for example falls by the person. Further refined algorithms may also be needed when a (declining) trend in activity is to be detected.

Embodiments of the present invention are therefore directed toward enabling an ADL of a person to be detected and/or monitored. This may be used to generate an alert signal for an alerting or warning system that can indicate the person is in need of help, for example.

Embodiments are based on the insight that the timing between detections of a person in two locations or areas can be used to infer a transition time and/or movement speed of a person, and from this the person's health or wellbeing may be assessed and/or monitored. Thus, instead of employing accelerometers and/or movement sensors and attempting to implement complex or computationally-intensive calculations on sensor data, proposed embodiments may obtain (e.g. receive) simple (e.g. binary) signals directly from presence detectors and then analyse such signals to determine a movement (e.g. walking) speed of a person. By using simple signals obtained from two presence detectors, embodiments may determine (e.g. infer) information about the physical well-being of a person without relying on complex and/or expensive sensor arrangements, thus reducing costs.

Proposed is a concept of inferring the movement speed of a person from the timing of changes or transitions in signals from two presence sensors. For example, a change or transition in a signal from a first sensor may indicate a person is no longer present in a first zone/area monitored by the first sensor. This may then be combined with a change or transition in a signal from a second sensor indicating a person has entered a second zone/area monitored by the second sensor. The time elapsed between the indication of the person leaving the first zone and entering the second zone may be used to determine a transition time of the person (i.e. a time taken for the person to transition between two zones/areas. The transition time may be analysed in conjunction with a known (or assumed) distance between the zone boundaries in order to calculate a speed of the person. By way of example, first and second sensor signals may be obtained from first and second PIR sensors, respectively, wherein the first PIR sensors is adapted to detect the presence of a person in a first room, and wherein the second PIR sensor is adapted to detect the presence of a person in a second, adjacent room.

Embodiments thus propose the use of the timings of detections from two presence sensors. An elapsed time between transitions/changes of the first and second sensors may be used to infer a movement speed of a person. Such a proposed speed inference system/method may therefore be employed in a system for monitoring a person within an environment.

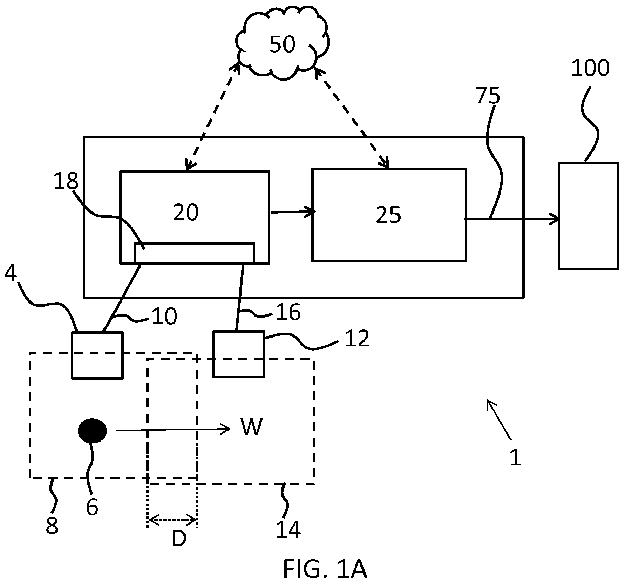

Fig. 1A shows an embodiment of a system 1 for monitoring a person in an environment (such as a home for example) according to an embodiment.

In this example, the environment includes a first sensor 4 adapted to detect the presence of a person 6 within a first zone 8 and to generate a first sensor output signal 10 representative of the detected presence/non-presence of a person 6 in the first zone 8. The environment also includes a second sensor 12 adapted to detect the presence of a person 6 within a second zone 14 and to generate a second sensor output signal 16 representative of the detected presence/non-presence of a person 6 in the second zone 14. In particular, in the example of Fig. 1A, each of the first 4 and second 12 sensors comprises a PIR sensor adapted output a binary signal indicating either presence or non-presence of a person in its monitored zone. Also, the first zone 8 overlaps the second zone 14 by a distance D. Put another way, the distance between adjacent boundaries of the first 8 and second 14 zone is the distance labelled "D" in Fig. 1A.

The first 4 and second 12 sensors communicate their respective sensor output signals 10, 16 to a signal interface 18 of a signal processing unit 20 of the system 1 via wired or wireless connections. The signal interface 18 is thus adapted to receive sensor output signals 10. 16 from the first 4 and second 12 sensors via one or more appropriately arranged communication links. By way of example, the wireless connections may comprise a short-to-medium-range communication link. For the avoidance of doubt, short-to-medium-range communication link should be taken to mean a short-range or medium-range communication link having a range of up to around 100 meters. In short-range communication links designed for very short communication distances, signals typically travel from a few centimetres to several meters, whereas, in medium-range communication links designed for short to medium communication distances, signals typically travel up to 100 meters. Examples of short-range wireless communication links are ANT+, Bluetooth, Bluetooth low energy, IEEE 802.15.4, ISA100a, Infrared (IrDA), ISM Band, Near Field Communication (NFC), RFID, 6LoWPAN, UWB, Wireless HART, Wireless HD, Wireless USB, ZigBee. Examples of medium-range communication links include Wi-Fi, Z-Wave.

The signal processing unit 20 is thus adapted to receive (via its signal interface 18) the first 10 and second 16 sensor output signals via one or more appropriately arranged communication links. Thus, it will be appreciated that the signal processing unit 20 may be remotely located from the monitored environment. In such an arrangement, the sensor signals 10,16 can be transferred to a local gateway, which processes and/or transmits the sensor signals to the remotely located signal processing unit 20 (e.g. using wired or wireless transmission, including cellular phone).

The signal processing unit 20 determines a value of time elapsed between a change in detected presence represented by the first sensor output signal 10 and a change in detected presence represented by the second sensor output signal 16. More specifically, in this example, the signal processing unit 20 is adapted to determine the value of time elapsed by determining the time elapsed between a change in the binary signal of first sensor output signal 10 and a change in the binary signal of the second sensor output signal 16.

By way of example, one may consider the circumstances depicted in Fig. 1A, wherein the person 6 is situated in the first zone 8 and then walks to the second 14 zone (as illustrated by the arrow labelled "W"). In such an example, as the person 6 walks from the first zone 8 to the second zone 14, the second sensor output signal 16 will indicate a change in detected presence (from no detected presence to detected presence). Also, the first sensor output signal 10 will subsequently indicate a change in detected presence (from detected presence to no detected presence). As will be seen from the illustration of Fig. 1A, because the first 8 and second 14 zones overlap, the first sensor output signal 10 will change/transition after the second output signal 16 changes/transitions (because the person 6 will enter the second zone 14 before leaving the first zone 8).

More specifically, the signal processing unit 20 may determine the time elapsed between:
(i) the second sensor output signal 16 from the second sensor 12 indicating a change in detected presence in the second zone 14 (i.e. from no detected presence of the person 6 to detected presence of the person 6); and
(ii) the first sensor output signal 10 from the first sensor 4 indicating a change in detected presence in the first zone 8 (i.e. from detected presence of the person 6 to no detected presence of the person).

Here, it is noted that, if one defines the transition time to relate to the time elapsed from the time that the first sensor output signal changes/transitions until the time that the second sensor output signal changes/transitions, the transition time will have a negative value for the example where the first 8 and second 14 zones overlap and the person 6 walks from the first zone 8 to the second zone 14 (i.e. the second sensor output signal changes/transitions before the first sensor output signal changes). Put another way, with the first 8 and second 14 zones overlapping, and the transition time being defined with respect to a transition in a particular direction or order (e.g. from first zone 8 to second zone 14), an obtained transition time may have a negative value.

Depending on configuration of the sensors and the direction in which a transition is defined, the transition time may have a positive or negative value. A direction of travel may thus be inferred from whether the transition time has a positive or negative value for example.

The value of time elapsed between the indication of the person 6 entering the second zone 14 and leaving the first zone 8 may then be analysed (in conjunction with the known distance D between the zone boundaries) in order to calculate the walking speed of the person 6. For this purpose, the signal processing unit 20 provides the determined value of elapsed time to a monitor unit 25.

The monitor unit 25 is adapted to determine a current value of the speed of the person 6 based on the value of elapsed time and the distance D between the zone boundaries. For this purpose, the monitor unit 25 may communicate with one or more data processing resources available in the internet or "cloud" 50. Such data processing resources may undertake part or all of the processing required to infer or determine a walking speed of the person 6 based on the obtained timings of transitions in the sensor output signals. Thus, the embodiment may employ distributed processing principles.

The monitor 25 is further adapted to generate an output signal 75 representative of determined walking speed of the monitored person 6. In other words, after determining a walking speed of the monitored person 6 (based on the obtained output signals 10,16 from the first 4 and second 12 sensors, either with or without communicating with data processing resources via the internet or "cloud"), an output signal 75 representative of a determined walking speed of the person is generated.

The system further comprises a graphical user interface (GUI) 100 for providing information to one or more users. The output signal 75 is provided to the GUI 100 via wired or wireless connection. By way of example, the wireless connection may comprise a short-to-medium-range communication link. As indicated in Fig. 1A, the output signal 75 is provided to the GUI 100 from the monitor unit 25. However, where the system, has made use of data processing resources via the internet or cloud 50, an output signal may be made available to the GUI 100 via the internet or cloud 50.

Based on the output signal 75, the GUI 100 is adapted to communicate information by displaying one or more graphical elements in a display area of the GUI 100. In this way, the system may communicate information about a walking speed of the monitored person that may be useful for indicating that the person is in need of attention or for estimating when the person may be expected to require assistance or attention. For example, the GUI 100 may be used to display graphical elements to a medical practitioner, a caregiver, a family member or close relative. Alternatively, or in addition, the GUI 100 may be adapted to display graphical elements to the monitored person 6.

Thus, the walking speed of the person 6 may be inferred from the relative timing of changes or transitions in the signals from first 4 and second 12 sensors. For instance, based on the determined value for the time elapsed between transitions in the first 10 and second 16 sensor output signals and a known (or assumed) distance between the monitored zones/areas, the walking speed of the person can be calculated by the monitor unit 25.

Put another way, it is proposed to obtain and analyse signals from two presence sensors. Since such presence sensors may be small or comprise pressure or weight sensors installed in floors of a monitored environment, embodiments may be completely unobtrusive and no additional sensors and/or monitors (such a light switch monitors, accelerometers, or light sensors, etc.) may be required in order to determine and monitor a movement speed of a person. The monitored person need only undertake their normal activities and may not even be aware that their movement is being monitored.

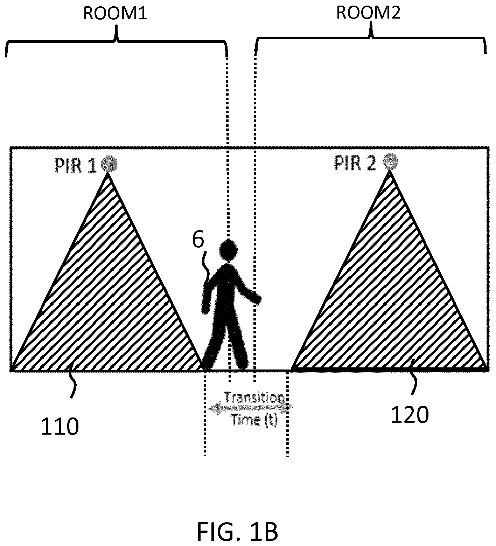

By way of further example, Fig. 1B illustrates an exemplary implementation according to a slightly modified version of the embodiment of Fig. 1A, wherein PIR sensors are implemented to detect the presence of person in first and second adjacent rooms.

More specifically, in the example of Fig. 1B, a first PIR sensor PIR1 is adapted to detect the presence of a person 6 within a first room ROOM1 and to generate a first sensor output signal representative of the detected presence/non-presence of a person 6 in the first room ROOM1. Also, a second PIR sensor PIR2 is adapted to detect the presence of a person 6 within a second room ROOM2 (adjacent to the first room ROOM1) and to generate a second sensor output signal representative of the detected presence/non-presence of a person 6 in the second room ROOM2. In particular, the coverage of the first PIR1 and second PIR sensors does not overlap. In other words, a first zone 110 monitored by the first PIR sensor PIR1 is separated or spaced apart from a second zone 120 monitored by the second PIR sensor PIR2.

In the example of Fig. 1A, each of the first PIR1 and second PIR2 sensors are adapted output a signal indicating either presence or non-presence of a person in its respective monitored zone. More specifically, an output signal is ON and transmitted by a sensor PIR1, PIR2 when the sensor detects presence of a person 6, and an output signal is OFF and not transmitted by a sensor PIR1, PIR2 when the sensor does not detect the presence of a person 6. Thus, it may be understood that sensor output signals received from the PIR sensors PIR1, PIR2 are either ON or OFF depending on whether or not the sensors detect the presence of a person 6 in their respective monitoring zones 110, 120. In this way, as the person 6 walks into the first room ROOM1, the sensor output signal from the first PIR sensor PIR1 will trigger ON (i.e. change from OFF to ON). Conversely, when the person 6 walks out of the first room ROOM1, the sensor output signal from the first PIR sensor PIR1 will trigger OFF (i.e. change from ON to OFF). Similarly, as the person 6 walks into the second room ROOM2, the sensor output signal from the second PIR sensor PIR2 will trigger ON (i.e. change from OFF to ON), and, when the person 6 walks out of the second room ROOM2, the sensor output signal from the second PIR sensor PIR2 will trigger OFF (i.e. change from ON to OFF).

Thus, if one considers the circumstances depicted in Fig. 1B, wherein the person 6 walks from the first room ROOM1 to the second room ROOM2, the second sensor output signal from the first PIR sensor PIR1 will trigger OFF (e.g. transition from 'have detected presence' to 'have not seen presence') to indicate a change in detected presence (from detected presence to no detected presence). Also, the sensor output signal from the second PIR sensor PIR2 will subsequently trigger ON to indicate a change in detected presence (from no detected presence to detected presence). As will be seen from the illustration of Fig. 1B, because the first 110 and second 120 monitored zones don't overlap, the sensor output signal from the first PIR sensor PIR1 will change/transition before the sensor output signal from the second PIR sensor PIR2 changes/transitions (because the person 6 will enter the second zone 120 after leaving the first zone 110). The value of time elapsed between the indication of the person 6 leaving the first zone 110 and entering the second zone 120 (i.e. the transition time (t)) may then be determined by comparing the timings of the changes/transitions in the output signals from the first PIR1 and second PIR2 sensors.

Knowing the separation distance D between the boundaries of the first 110 and second 120 zone and the transition time (t), the walking speed of the person 6 may then be calculated.

Nonetheless, as mentioned above, other embodiments need not determine the walking speed of the person, but may instead simply determine the transition time (t).

When combining measurements from different room transitions the distance D can be used to normalize the measured transition times. However, another way may be to normalize by using the mean or other central value (e.g., median and mode) of the observed transition times for each room combination (including per direction of each pair). One may view this normalization as a virtual distance D.

Such use of data from presence sensors may ensure that movement of the monitored person between two areas or rooms can be automatically and accurately obtained without requiring the person to remember to undertake any special or additional activities in order for movement between the areas/rooms to be detected. For example, it can remove the need for a person to perform a specific additional action (e.g. pressing a button) in order to indicate or log activity.

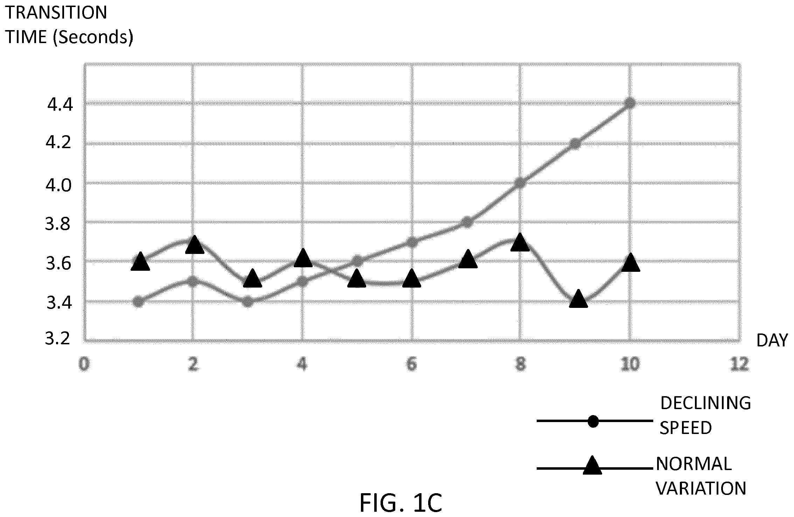

From the above description of the embodiments of Figure 1, it will be understood that there is proposed a system which analyses the relative timings of transitions in signals from two presence sensors so as to determine a movement speed of a monitored person. This may be thought of as providing speed detection and/or monitoring on a short term timescale (for example, at real-time). Such implementations may, for example, detect current speeds and potentially identify recommended actions. For instance, if the monitored person determined as too slow (as may be inferred from a time between transitions in the two sensor output signals being longer than a predefined threshold time (e.g. 10 seconds, 30 seconds, 1 minute or alternatively if twice as long as average of previous 5 times)), an alert signal may be provided to caregiver. Here, it is noted that it may be preferable to define the threshold to be somewhat close to a normal/expected transition time in an attempt to avoid outliers or anomalies being captured and/or considered. Also, embodiments may be adapted to request that the monitored person acknowledges the alert before an alert signal is provided to the caregiver (so as to provide an opportunity for the person to indicate the alarm is a false alarm for example).

Other embodiments, however, may provide transition time detection and/or monitoring on a long term timescale (e.g. for pattern recognition and trend determination). Such implementations may, for example, obtain information about activities of the person, e.g. activity/movement levels or patterns. Also, deviations from usual patterns or trends may be detected and translated into an alert or alarm. Such deviations may relate to incidental deviations (e.g. user did not go to bed during the night) or indicate gradually increasing deviations (e.g. indication of cognitive decline or physical decline).

Thus, some embodiments may further comprise a monitor unit adapted to determine a trend in a capability or activity of the person based on a determined current transition time and historical data relating to previously determined transition times of the person.

Accordingly, proposed is a concept for monitoring a person by determining a trend in the transition time of the person.

In general, to be able to observe trends in "normal" daily behaviour of a person one may monitor the movement and transition time of a person. From established trends, one may also spot unexpected activities, anomalies or deviations from expected values or patterns. The type of anomaly or irregularity can be different per case.

A large class of physical or mental capabilities can relate to an ADL routine of the person. For example, a physical capability may be inferred from the speed by which a person moves between rooms. Also, the transitions between rooms/areas may provide a measurement of total physical effort, which can be useful for monitoring purposes. Embodiments of the present invention may therefore be directed toward enabling information about a physical or mental capability of a person to be obtained and potentially monitored. Such information may therefore be useful for monitoring the health or well-being of a person.

Some embodiments can employ a concept of determining a trend in a (physical or mental) capability or activity of a person from a currently sensed or determined transition time of the person and historical data relating to one or more previously detected speeds of the person. In other words, determination of a trend in a capability or activity of a person may be based on current and previously detected transition times of the person. Such a proposed concept for monitoring a physical or mental capability or activity of a person may therefore be employed in a system for monitoring ADLs of a person within an environment.

For the purpose of such long-term monitoring and/or trend identification, determined transition times (and associated values) may be stored in a database adapted to store historical data relating to one or more previously detected transitio times. In doing so, each determined transition time value may be labelled with the timestamp identifying when the value was measured. In the trend determination or analysis, the detected values may then be averaged per time unit, e.g. per day or per day portion, before the trend is estimated. Also, given a (known) context, one may also choose to exclude or include certain days. For example, on Fridays, the grandchildren or a cleaning lady are in the house and so detected values may not be representative of the monitored person.

Another approach may be to provide each detected transition time value event as a pair of time/date and value, so that trend analysis, using a regression method for example, may account for the varying rate (e.g. irregularity in arrival) at which values are detected.

By storing previous detected transition time values, estimates of a trend in the transition times may be determined and a future date/time at which the person may require help or assistance may be identified (e.g. by extrapolating the trend to identify when it goes above/below a threshold value). Furthermore, currently detected transition times may be used to re-calculate or refine previously determined trends (e.g. those stored in a database for example). Further, embodiments may be self-learning (e.g. using machine learning algorithms) by using previously detected values.

By way of example, Fig. 1 C illustrates a graph depicting transition time (in seconds) against (numbered) consecutive days for a normal variation/trend (depicted by the line marked with triangles) and for a trend of declining speed (depicted by the line marked with circles). As will be seen, the trend of increasing transition time is apparent as the transition instances increase.

When analysing such trends, it may be preferable to apply Dynamic Time Warping (DTW) or similar techniques to normalize the time scale. Also, embodiments may only observe (or weight) selected transitions (e.g. transitions between particular room/zone pairs and/or transitions in a particular time period of a day). Weighting can be to both contribution in the overall estimate, as well as in normalizing the time scale. For example, during sleep (night) other typical/expected walking speeds may apply, so that another time scale may be employed when comparing with day-time transition times.

Although the embodiments of Figs. 1A and 1B employ signals from PIR sensors, it will be understood that many other different types of presence sensors/detectors may be employed to detect the presence of a person in different areas. For example, other embodiments may employ optical sensors, such as cameras and coded light, radiation-based sensors such as WiFi (signal strength with phone, or absorption in radiation pattern by human body), radar, pressure sensors or weight sensors adapted to detect the presence of a person within respective areas.

By detecting transition times based on relative timings of transitions or changes in signals from multiple presence sensors that a person triggers, a trend in the detected transition times over time may be identified and, from such a trend, a capability or activity of a person may be monitored. For example, a trend of increasing transition times or slowing speeds (e.g. increasing time between triggering of two presence sensors) may be used to identify and monitor a trend in the person's physical or cognitive abilities.

Proposed embodiments may therefore reduce requirements on the placement of the sensors. For example, there may be no need for dedicated or exact locations of the sensors so that they have specific inter-sensor distances. For instance, rather than focussing on obtaining an exact or accurate speed measurement for a single transition between two zones, embodiments may instead observe a trend or pattern in the transition times (and thus the corresponding walking speed). With the inter-sensor distance remaining constant, it may be a trend or pattern in transition times that provides information of interest (rather than the exact speed of a single isolated transition).

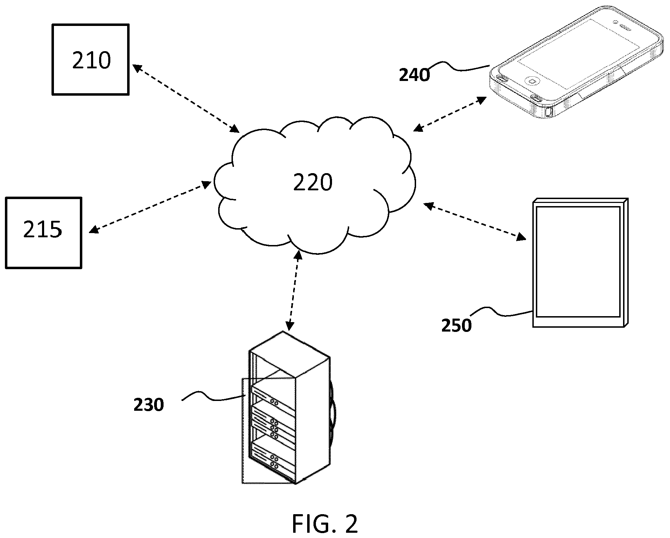

Referring now to Fig. 2, there is depicted another embodiment of a system according to the invention comprising first 210 and second 215 presence sensors. Here, the first presence sensor 210 is adapted to detect the presence of a person within a first room and to generate a first sensor output signal representative of the detected presence of a person in the first room. The second presence sensor 215 is adapted to detect the presence of a person within a second room (that is adjacent to and separate from the first room) and to generate a second sensor output signal representative of the detected presence of a person in the second room.

The first 210 and second 215 presence sensors are each adapted to communicate their respective sensor output signals (e.g. signals indicating detected presence or non-presence of person) via the internet 220 (using a wired or wireless connection for example) to a remotely located signal processing system 230 (such as server).

The signal processing system 230 is adapted to receive the sensor output signals from the first 210 and second 215 presence sensors and process the received sensor output signals in accordance with a timing detection algorithm in order to determine a value of time elapsed between a change in detected presence represented by the first sensor output signal and a change in detected presence represented by the second sensor output signal. Based on the determined value of elapsed time the signal processing system then determines a current value of the speed of the person.

The signal processing system is further adapted to processes the current value of the speed of the person in combination with historical data relating to one or more previously determined speeds of the person to determine a trend in the speed over time. The trend can be found as a linear line (e.g., using linear regression), but may also be curved using higher order fitting techniques.

The data processing system 230 is further adapted to generate output signals representative of inferred or calculated speed trend. Thus, the data processing system 230 provides a centrally accessible processing resource that can receive information from the presence sensors 210,215 and run one or more algorithms to transform the received information into a description of a trend in a speed of the person.

Previously determined speed values (and associated information, such as time, date, description, etc.) may therefore be stored, in a historical database for example, and then used in subsequent calculations. Furthermore, currently detected speed value may be used to re-calculate or refine a previously determined trend. Thus, the pattern of speed and/or movement of the person may be stored. Shifts in this pattern may indicate that the person is in need of help. For example, the person may start forgetting to take a regular shower, and this may be inferred from a trend in a frequency of movement to a particular room (e.g. shower room) of the monitored home.

Further, the data processing system 230 is adapted to detect an irregularity in the historical database. For example, by adding time information to the determined speed(s), the time spent between the movements may be determined. With the time information, the frequency of the movement may be determined. An irregularity in the historical database may then, for example, be that the time spent between the determined activities of 'moving to Room 2' has increased.

Such provision of information about a detected or inferred speed/movement trend and/or irregularity may be undertaken in response to a receiving a request (via the internet 220 for example) and/or may be undertaken without request (i.e. 'pushed').

For the purpose of receiving information about a detected speed trend from the data processing system, and thus to enable the person to be monitored, the system further comprises first 240 and second 250 mobile computing devices.

Here, the first mobile computing device 240 is a mobile telephone device (such as a smartphone) with a display for displaying graphical elements representative of a person's physical or mental well-being. The second mobile computing device 250 is a mobile computer such as a Laptop or Tablet computer with a display for displaying graphical elements representative of a person's speed.

The data processing system 230 is adapted to communicate output signals to the first 240 and second 250 mobile computing devices via the internet 220 (using a wired or wireless connection for example). As mentioned above, this may be undertaken in response to receiving a request from the first 240 or second 250 mobile computing devices.

Based on the received output signals, the first 240 and second 250 mobile computing devices are adapted to display one or more graphical elements in a display area provided by their respective display. For this purpose, the first 240 and second 250 mobile computing devices each comprise a software application for processing, decrypting and/or interpreting received output signals in order to determine how to display graphical elements. Thus, the first 240 and second 250 mobile computing devices each comprise a processing arrangement adapted to one or more values representative of a trend, and to generate a display control signal for modifying at least one of the size, shape, position, orientation, pulsation or colour of the graphical element based on the one or more values representative of trend.

The system can therefore communicate information about a detected speed of a monitored person to users of the first 240 and second 250 mobile computing devices. For example, each of the first 240 and second 250 mobile computing devices may be used to display graphical elements to a medical practitioner, a caregiver, a family member or close relative. Also, the system can generate a warning signal in response to a detected irregularity. The irregularity may, for example, indicate that the person is in need of help and thus a generated alert or warning signal may be provided to at least one of: the person; a medical practitioner; and a caregiver.

Implementations of the system of Figure 2 may vary between: (i) a situation where the data processing system 230 communicates display-ready speed trend data, which may for example comprise display data including graphical elements (e.g. in JPEG or other image formats) that are simply displayed to a user of a mobile computing device using conventional image or webpage display (which can be web based browser etc.); to (ii) a situation where the data processing system 230 communicates raw data set information that the receiving mobile computing device then processes to determine a trend in a person's speed, and then displays graphical elements based on the determined trend (for example, using local software running on the mobile computing device). Of course, in other implementations, the processing may be shared between the data processing system 230 and a receiving mobile computing device such that part of the data generated at data processing system 230 is sent to the mobile computing device for further processing by local dedicated software of the mobile computing device. Embodiments may therefore employ server-side processing, client-side processing, or any combination thereof.

Further, where the data processing system 230 does not 'push' information (e.g. output signals), but rather communicates information in response to receiving a request, the user of a device making such a request may be required to confirm or authenticate their identity and/or security credentials in order for the information to be communicated.

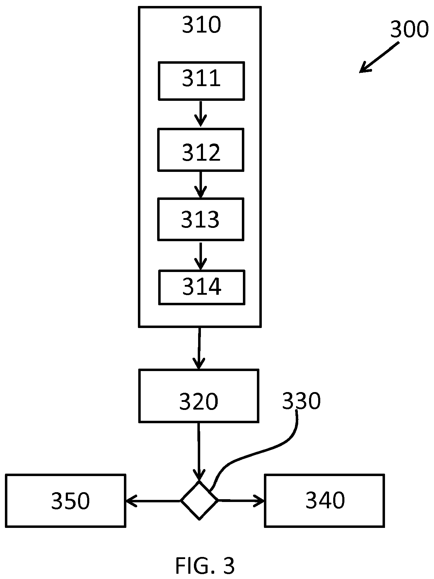

Referring now to Figure 3, there is shown a flow diagram of an exemplary method 300 for monitoring a person.

The method begins with step 310 in which a walking speed of the person is detected. Specifically, the step 310 of detecting a speed of a person comprises the sub-steps of: detecting 311, with a first sensor, the presence of a person within a first zone and generating a first sensor output signal representative of the detected presence; detecting 312, with a second sensor, the presence of a person within a second zone and generating a second sensor output signal representative of the detected presence; determining 313 a value of time elapsed between a change in detected presence (being an end presence) represented by the first sensor output signal and a change in detected presence (being a start of presence) represented by the second sensor output signal; and determining 314 a current value of the transition time of the person based on the determined value of time.

Next, in step 320, a trend in transition times of the person is determined based on the current value of the transition time of the person that was determined in step 314 and historical data relating to one or more previously detected transition times (of the monitored person).

Then, in step 330, the determined trend is compared with a predetermined threshold value. The threshold value can be pre-programmed, fixed or dynamically set in response to calculations based on one or more previously obtained transition time values (e.g. using trend analysis), but is preferably also enabled to be set by a user preference. Thus, the threshold may be based on previously determined values representative of a physical or mental capability the person. In other words, the threshold may be defined by taking account of a history of the person and/or taking account of previous calculations so that it can be used to identify outlying values or anomalies.

If, in step 330, the trend is determined to exceed the first threshold value, the method proceeds to step 340 wherein a warning signal is generated and output along with information describing the trend and/or the detected transition time. If, in step 330, the trend is determined to not exceed the first threshold value, the method proceeds to step 350 wherein the information describing the detected transition time value is output and/or stored without any warning signal.

Here, it is noted that in alternative embodiments, step 330 may also perform another test procedure, e.g. using the CUSUM algorithm. This may bypass the trend computation in step 320.

From the above description of the method illustrated by Figure 3, it will be understood that proposed embodiments may be adapted to detect an irregularity in a determined trend. For example, in the embodiment of Figure 3, the steps of 330 to 350 may be implemented by a monitor unit and be implemented to detect an irregularity based on a comparison of a determined trend with a threshold value. This may provide the advantage that an alarm or alert signal may be generated and communicated when an irregularity or anomaly is detected (e.g. if the person takes too long to walk between two adjacent rooms, indicating that the person may have become too unwell/weak). In this example, to detect an irregularity, the monitor unit undertakes a relatively simple comparison of the determined trend with a threshold value. The threshold may be preprogramed and fixed, but it may be preferable to enable the threshold value to be set by a user preference. This may identify when a threshold value may be exceeded in the future, for example.

Other methods to detect outliers or to detect changes in a time series are known in the art. By way of example, in an embodiment, the monitor unit may be further adapted to calculate an estimated future value of the property based on the determined trend, and the monitor unit may be further arranged to detect an irregularity based on a comparison of the estimated future value of the property with a threshold value and to generate an alert output signal in response to the detected irregularity. A threshold value may thus relate to a future value, and a determined trend may be extrapolated for comparison of an extrapolated value with the future threshold value. Using the extrapolated trend, one may estimate the time until the trend will cross the threshold, and provide that number as an identification of when intervention or assistance may be required, for example. In another embodiment, the trend may simply be displayed to user and left for visual inspection (e.g. for the purpose of identifying outliers or changes in a trend).

Purely by way of example, one or more steps of the method 300 for monitoring a physical or mental capability of a person may be implemented in a portable computing device (such as the smartphone or portable computer shown in Figure 2) in order to control the display of graphical elements on a display. Of course, it will be understood that proposed embodiments for monitoring a person's transition times may be implemented in other methods and/or systems.

Embodiments may further comprise a control unit adapted to generate one or more control signals for the control system based on the determined transition time value. In this way, embodiments may be adapted to provide control signals that are suitable for use by a control system. Accordingly, rather than just monitoring transition times of a person, some embodiments may be adapted to operate a control system so as to assist the person. For example, if movement between two rooms is detected, and if it happens in low light, an embodiment may communicate a control signal to the control system which causes the control system to change or improve the lighting conditions (e.g. automatically switch off the light in the room being left and activate one or more devices at a projected time of arrival based on the detected transition time of the person).

It is also noted that, although it has been described above that embodiments need not employ additional/supplementary sensors, some embodiments may further comprise a sensor adapted to detect a value of a property of at least one of: the environment, control/operation of an object, and the monitored person. Such a supplementary sensor arrangement may help to improve the accuracy of speed determination for example. Supplementary sensor readings may, for instance, qualify or refine data analysis undertaken by the signal processing unit and/or the monitor unit. For instance, the person may wear or carry an identification tag or location tracker which can enable embodiments to distinguish and disregard the presence of other people or animals detected by the presence sensors. By way of further example, signals from a location sensor worn by the monitored person may be used to confirm if presence detections are indeed attributable to the monitored person or some other person.

The presence sensors may be arranged in strategic locations/positions so as to detect a person's presence without the person needing to intentionally or consciously activate/operate the sensors. In this way, a person may only need to undertake their normal activities. Such strategic positioning may ensure that presence of the person can be automatically and accurately detected, and this may not require the person to remember to undertake any special or additional activities in order for a value to be detected by the sensor. This may remove the risk of the person forgetting to activate a sensor (e.g. by pressing a button), for example.

There exist many sensors that can be employed by embodiments. Typical sensors include PIR (pyroelectric infrared sensor; detect movement and presence) or pressure sensors. Many others exist and are conceivable, such as sensors comprising microphones and cameras (including infra-red (IR), or even UV and beyond, part of spectrum).

The sensors may also be adapted to undertake primary processing of the detected values, such a signal filtering, sampling, conditioning, etc., so as to reduce a required transmission bandwidth and/or transmission duration for example.

Non-intrusive monitoring may therefore be realized with relatively simple sensors that provide data on specific properties of the person (such as movement, for example). Also, the movement of the person may be detected with sensors that are cheap and widely employed. For instance, movement sensors may be used to switch on lighting and people are therefore typically familiar with their usage. Thus, embodiments may employ sensors that are considered to be non-intrusive and more easily accepted by the monitored person. Yet, with the data provided by these sensors, a person's speed may be accurately determined and provide more information on the person being monitored. Thus, some embodiments of the invention may employ conventional sensors and/or existing sensor arrangements. Also, embodiments may employ sensors that are considered to be non-intrusive and more easily accepted by the monitored person.

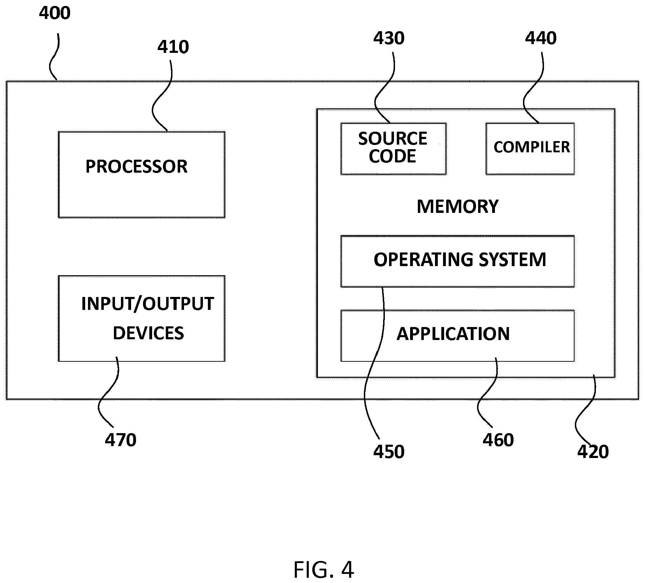

Figure 4 illustrates an example of a computer 400 within which one or more parts of an embodiment may be employed. Various operations discussed above may utilize the capabilities of the computer 400. For example, one or more parts of a monitoring system adapted to monitor a person's speed may be incorporated in any element, module, application, and/or component discussed herein.

The computer 400 includes, but is not limited to, PCs, workstations, laptops, PDAs, palm devices, servers, storages, and the like. Generally, in terms of hardware architecture, the computer 400 may include one or more processors 410, memory 420, and one or more I/O devices 470 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 410 is a hardware device for executing software that can be stored in the memory 420. The processor 410 can be virtually any custom made or commercially available processor, a central processing unit (CPU), a digital signal processor (DSP), or an auxiliary processor among several processors associated with the computer 400, and the processor 410 may be a semiconductor based microprocessor (in the form of a microchip) or a microprocessor.

The memory 420 can include any one or combination of volatile memory elements (e.g., random access memory (RAM), such as dynamic random access memory (DRAM), static random access memory (SRAM), etc.) and nonvolatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 420 may incorporate electronic, magnetic, optical, and/or other types of storage media. Note that the memory 420 can have a distributed architecture, where various components are situated remote from one another, but can be accessed by the processor 410.

The software in the memory 420 may include one or more separate programs, each of which comprises an ordered listing of executable instructions for implementing logical functions. The software in the memory 420 includes a suitable operating system (O/S) 450, compiler 440, source code 430, and one or more applications 460 in accordance with exemplary embodiments. As illustrated, the application 460 comprises numerous functional components for implementing the features and operations of the exemplary embodiments. The application 460 of the computer 400 may represent various applications, computational units, logic, functional units, processes, operations, virtual entities, and/or modules in accordance with exemplary embodiments, but the application 460 is not meant to be a limitation.

The operating system 450 controls the execution of other computer programs, and provides scheduling, input-output control, file and data management, memory management, and communication control and related services. It is contemplated by the inventors that the application 460 for implementing exemplary embodiments may be applicable on all commercially available operating systems.

Application 460 may be a source program, executable program (object code), script, or any other entity comprising a set of instructions to be performed. When a source program, then the program is usually translated via a compiler (such as the compiler 440), assembler, interpreter, or the like, which may or may not be included within the memory 420, so as to operate properly in connection with the O/S 450. Furthermore, the application 460 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, php. Python, ASP scripts, FORTRAN, COBOL, Perl, Java, ADA, .NET, and the like.

The I/O devices 470 may include input devices such as, for example but not limited to, a mouse, keyboard, scanner, microphone, camera, etc. Furthermore, the I/O devices 470 may also include output devices, for example but not limited to a printer, display, etc. Finally, the I/O devices 470 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 470 also include components for communicating over various networks, such as the Internet or intranet.

If the computer 400 is a PC, workstation, intelligent device or the like, the software in the memory 420 may further include a basic input output system (BIOS) (omitted for simplicity). The BIOS is a set of essential software routines that initialize and test hardware at startup, start the O/S 450, and support the transfer of data among the hardware devices. The BIOS is stored in some type of read-only-memory, such as ROM, PROM, EPROM, EEPROM or the like, so that the BIOS can be executed when the computer 400 is activated.

When the computer 400 is in operation, the processor 410 is configured to execute software stored within the memory 420, to communicate data to and from the memory 420, and to generally control operations of the computer 400 pursuant to the software. The application 460 and the O/S 450 are read, in whole or in part, by the processor 410, perhaps buffered within the processor 410, and then executed.

When the application 460 is implemented in software it should be noted that the application 460 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 460 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The present invention may be a system, a method, and/or a computer program product. The computer program product may include a computer readable storage medium (or media) having computer readable program instructions thereon for causing a processor to carry out aspects of the present invention.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present invention may be assembler instructions, instruction-set-architecture (ISA) instructions, machine instructions, machine dependent instructions, microcode, firmware instructions, state-setting data, or either source code or object code written in any combination of one or more programming languages, including an object oriented programming language such as Java, Smalltalk, C++ or the like, and conventional procedural programming languages, optimized for embedded implementation, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present invention.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

From the above description, it will be appreciated that embodiments propose to use two or more presence sensors for purposes of detecting and monitoring a movement (e.g. walking) speed of a person. Embodiments may therefore be useful for monitoring of elderly, disabled or unwell individuals so to support independent living. Usage data from the control system can be used both for real-time speed detection and alerts, as well as to detect gradual deviations from usual patterns or trends.

Embodiments may be supported by the current and future trends towards smart-homes or connected lighting.

The description has been presented for purposes of illustration and description, and is not intended to be exhaustive or limited to the invention in the form disclosed. Many modifications and variations will be apparent to those of ordinary skill in the art. Embodiments have been chosen and described in order to best explain principles of proposed embodiments, practical application(s), and to enable others of ordinary skill in the art to understand that various embodiments with various modifications are contemplated.

## Claims

1. A system for monitoring activity of a person, wherein the system comprises:
a signal interface (18) adapted to receive: a first sensor output signal (10) representative of detected presence of a person (6) within a first zone (8); and a second sensor output signal (16) representative of detected presence of a person (6) within a second zone (14);
a signal processing unit (20) adapted to determine a value of time elapsed between a change in detected presence represented by the first sensor output signal and a change in detected presence represented by the second sensor output signal; and
a monitor unit (25) adapted to determine a current value of a transition time of the person based on the determined value of time.

2. The system of claim 1, further comprising:
a first sensor (4) adapted to detect the presence of a person (6) within the first zone (8) and to generate the first sensor output signal (10) representative of the detected presence; and
a second sensor (12) adapted to detect the presence of a person within the second zone (14) and to generate the second sensor output signal (16) representative of the detected presence.

3. The system of claim 2, wherein each of the first (4) and second sensors (12) comprise at least one of:
a camera;
a radar system;
a WiFi signal detection system;
a pyroelectric infrared sensor;
a weight sensor; and
a pressure sensor.

4. The system of claim 2 or 3, wherein the monitor unit (25) is remotely located from the first (4) and second (12) sensors, and wherein the first and second sensor output signal are communicated to the monitor unit via respective communication links.

5. The system of any preceding claim, wherein each of the first (10) and second (16) sensor output signals comprises a binary signal indicating either detected presence or detected non-presence of the person, and wherein the signal processing unit (20) is adapted to determine the value of time by determining the time elapsed between a change in the binary signal of first sensor output signal and a change in the binary signal of the second sensor output signal.

6. The system of any preceding claim, wherein the monitor unit (25) is further adapted to determine a trend in the transition time of the person based on the current value of the transition time of the person and historical data relating to one or more previously determined values of the transition time of the person.

7. The system of claim 6, wherein the monitor unit (25) is further adapted to detect an anomaly in the determined trend, and wherein the monitor unit is further arranged to generate an alert output signal in response to the detected anomaly

8. The system of claim 7, wherein the monitor unit (25) is adapted to detect an anomaly based on a comparison of the determined trend with a threshold value, the threshold value, and preferably wherein the threshold value is determined based on at least one of:
one or more previously detected values of the transition time of the person;
and
one or more previously determined changes in values of the transition time of the person.

9. The system of any preceding claim, wherein the first zone (8) comprises a first room of a property and wherein the second zone (14) comprises a second room of the property, the second room being adjacent to the first room.

10. The system of any preceding claim, wherein the monitor unit (25) is further adapted to calculate an estimated future value of the property based on the determined trend, and wherein the monitor unit is further arranged to detect an anomaly based on a comparison of the estimated future value of the property with a threshold value and to generate an alert output signal in response to the detected irregularity.

11. The system of any preceding claim, wherein the monitor unit (25) is further adapted to generate a control signal for modifying a graphical element based on the determined trend in the physical or mental capability of the person,
and wherein the system further comprises:
a display system adapted to display the graphical element in accordance with the control signal generated by the monitor unit.

12. A method for monitoring activity of a person, wherein the method comprises:
detecting (311), with a first sensor, the presence of a person within a first zone
and generating a first sensor output signal representative of the detected presence;
detecting (312), with a second sensor, the presence of a person within a second zone and generating a second sensor output signal representative of the detected presence;
determining (313) a value of time elapsed between a change in detected presence represented by the first sensor output signal and a change in detected presence represented by the second sensor output signal; and
determining (313) a current value of a transition time of the person based on the determined value of time.

13. The method of claim 12, wherein each of the first and second sensor output signals comprises a binary signal indicating either detected presence or detected non-presence of the person,
and wherein determine the value of time comprises determining the time elapsed between a change in the binary signal of first sensor output signal and a change in the binary signal of the second sensor output signal.

14. The method of claim 12 or 13, further comprising:
determining (320) a trend in the physical or mental capability of the person based on the current value of the transition time of the person and historical data relating to one or more previously determined values of the transition time of the person,
and optionally further comprising:
detecting an anomaly in the determined trend; and
generating an alert output signal in response to the detected anomaly.

15. A computer program product comprising computer readable code storable on, or stored on, or downloadable from a communications network, which code when run on a computer implements any one of the methods of claims 12 to 14.
